# EUROPEAN PATENT APPLICATION

(11) **EP 4 552 593 A1**
(43) Date of publication of application: **14.05.2025**
(21) Application number: 23835373.4
(22) Date of filing: 27.06.2023
(51) Int. Cl.: A61B 18/14

(54) **NEEDLE BASE COVER AND HIGH-FREQUENCY TREATMENT DEVICE**

(30) Priority: 04.07.2022 JP 2022107759
(71) Applicant: Kabushiki Kaisha Top, Adachi-ku, Tokyo 120-0035 (JP)
(72) Inventor: MURATA, Koji, Tokyo 120-0035 (JP); MIYAZAWA, Yoshihiro, Tokyo 120-0035 (JP)
(74) Representative: Schön, Christoph
(86) International application number: PCT/JP2023/023731
(87) International publication number: WO 2024/009840

(57) **Abstract**

Provided are a needle base cover capable of stabilizing relative positions and postures of a plurality of electrode needles and a high-frequency treatment device having a plurality of electrode needles of which proximal end portions are protected by the needle base cover. A needle base cover 1 includes a base portion 10, a first clamping portion 11 and a second clamping portion 12 as a pair of clamping portions. An inner side portion of the first clamping portion 11 has a first uneven structure 110 that extends in a waveform shape in a Z direction (first designated direction) while undulating in an X direction (designated direction). An inner side portion of the second clamping portion 12 has a second uneven structure 120 that extends in a waveform shape in a Y direction (second designated direction) while undulating in the X direction. One outer side portion of the base portion 10 has a first sub-uneven structure 140 that extends in a waveform shape in the Z direction while undulating in the X direction. Other outer side portion of the base portion 10 has a second sub-protruding portion 144 that protrudes in the X direction.

## Description

### Technical Field

The present invention relates to a high-frequency treatment device that applies a high-frequency voltage to a treatment target such as a human or an animal and performs a treatment such as thermal coagulation of a nerve tissue and/or cauterization of a tumor tissue, and a needle base cover that protects a proximal end portion of an electrode needle used in the high-frequency treatment device.

### Background Art

In the related art, in the medical field, a high-frequency treatment of puncturing an electrode needle in a body of a human or another animal and causing a high-frequency current to flow into the body from the electrode needle to cauterize (ablate) tissues has been performed. A nerve block by the high-frequency treatment, which is one of pain curing methods, has an advantage that side effects on tissues around a treatment tissue are small and effects last for a long period of time, as compared with a method of a pain treatment using a medicine.

There are two types of high-frequency treatment of a high-frequency thermal coagulation method and a pulsed high-frequency method, for the nerve block. The high-frequency thermal coagulation method is a method of heating a part of the nerve tissue at a temperature of about 80°C for several minutes by high-frequency power output from the electrode needle to thermally coagulate the nerve tissue, thereby blocking a pain signal. The pulsed high-frequency method is a method of heating a part of the nerve tissue at a temperature of 42°C or lower for over ten minutes by high-frequency power intermittently output from the electrode needle and blocking a pain signal without damaging the nerve (for example, see Patent Literature 1).

### Citation List

### Patent Literature

Patent Literature 1: PCT Japanese Translation Patent Publication No. 2018-511444

### Summary of Invention

### Technical Problem

However, in a state where a plurality of electrode needles punctures a treatment target such as a human body, relative positions and postures of the plurality of electrode needles may be unstable or difficult to adjust. This is particularly remarkable in a state where the plurality of electrode needles are punctured close to each other with respect to the treatment target.

Therefore, an object of the present invention is to provide a needle base cover capable of stabilizing relative positions and postures of a plurality of electrode needles and a high-frequency treatment device having a plurality of electrode needles of which proximal end portions are protected by the needle base cover.

### Solution to Problem

A needle base cover according to the present invention is a needle base cover for protecting a proximal end portion of an electrode needle that outputs high-frequency power to a treatment target in a state of being punctured to the treatment target, the needle base cover including a base portion that has a pair of outer side portions which are on opposite sides to each other in a designated direction, and in which a through-hole extending in a first designated direction perpendicular to the designated direction in which the proximal end portion of the electrode needle is inserted is formed; and a pair of clamping portions that extend from the base portion in a second designated direction perpendicular to each of the designated direction and the first designated direction, and face each other in the designated direction, in which one side portion of one outer side portion of the pair of outer side portions and an inner side portion of one clamping portion of the pair of clamping portions of the base portion has a first uneven structure extending in a waveform shape in the first designated direction, and the other side portion has a first sub-protruding portion protruding in the designated direction, and one side portion of the other outer side portion of the pair of outer side portions and an inner side portion of the other clamping portion of the pair of clamping portions of the base portion has a second uneven structure extending in a waveform shape in the second designated direction, and the other side part has a second sub-protruding portion protruding in the designated direction.

A high-frequency treatment device according to the present invention including a plurality of needle base covers according to the present invention; a plurality of electrode needles of which a proximal end portion is protected by each of the plurality of needle base covers; and a plurality of electrode cables that are electrically connected to the proximal end portion of each of the plurality of electrode needles, in which the high-frequency treatment device is configured such that electrical connection is performed in each of the plurality of electrode needles via the plurality of electrode cables, and a high-frequency voltage is applied between the plurality of electrode needles.

### Brief Description of Drawings

FIG. 1 is a schematic explanatory view of a configuration of a high-frequency treatment device.
FIG. 2 is a top view of a needle base cover according to one embodiment of the present invention.
FIG. 3 is a first perspective view of a needle base cover according to one embodiment of the present invention.
FIG. 4 is a second perspective view of the needle base cover according to one embodiment of the present invention.
FIG. 5 is a third perspective view of the needle base cover according to one embodiment of the present invention.
FIG. 6 is a fourth perspective view of the needle base cover according to one embodiment of the present invention.
FIG. 7 is an explanatory view of a relative displacement aspect of the needle base cover in a first designated direction.
FIG. 8 is an explanatory view of a relative displacement aspect of the needle base cover in the first designated direction.
FIG. 9 is an explanatory view of a relative displacement aspect of the needle base cover in a second designated direction.
FIG. 10 is an explanatory view of a relative displacement aspect of the needle base cover in the second designated direction.

### Description of Embodiments

### (Configuration of high-frequency treatment device)

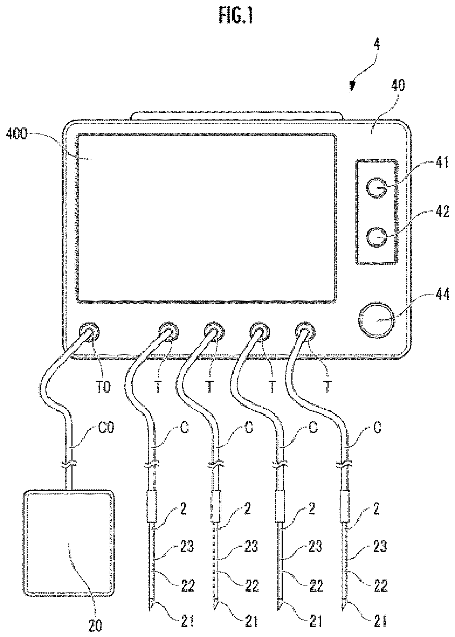

A high-frequency treatment device 4 according to a first embodiment of the present invention illustrated in FIG. 1 is a device for performing a nerve block by partially heating a peripheral nerve with a high-frequency current. As illustrated in FIG. 1, the high-frequency treatment device 4 includes a main body 40 (housing or casing), four electrode needles 2 connected to the main body 40, and a counter electrode plate 20 connected to the main body 40. The number of electrode needles 2 may be "2", "3", or "5 or more" instead of "4" in a case where the number of electrode needles 2 is plural.

As an output format of high-frequency power for the treatment target, four types of output formats of a monopolar output format, a bipolar output format, a tripolar output format, and a quadrupolar output format can be selected. The monopolar output format is an output format in which a high-frequency voltage is applied (or a high-frequency current flows) between a single electrode needle 2 and the counter electrode plate 20. The bipolar output format is an output format in which the high-frequency voltage is applied between two electrode needles 2. The tripolar output format is an output format in which the high-frequency voltage is applied between one electrode needle 2 and the two electrode needles 2. In the quad output format, for example, the high-frequency voltage is applied between the electrode needles 2 forming a first pair and between the electrode needles 2 forming a second pair. This is an output format in which a combination of the electrode needles 2 forming a pair is periodically switched between a case where the high-frequency voltage is applied to the electrode needles 2 forming the first pair and a case where the high-frequency voltage is applied to the electrode needles 2 forming the second pair.

The main body 40 is configured to accommodate or support configuration elements, which will be described below. Each of four electrode connectors T to which four electrode needles 2 is electrically connected and a counter electrode plate connector T0 to which the counter electrode plate 20 is electrically connected are provided at a lower portion of a front surface of the main body 40.

A touch panel display 400 that receives an input operation of various settings and the like, and displays various types of information, an ON button 41 that receives a treatment start operation, an OFF button 42 that receives a treatment end operation, and a control knob 44 for adjusting output power are provided on the front surface of the main body 40. A target temperature and/or a target output power from the electrode needle 2 (a target voltage value and/or a target current value) can be set for each of the electrode needles 2 through the control knob 44 and/or the touch panel display 400. A rear surface of the main body 40 is provided with a power supply connector and a power supply switch that are connected to a commercial AC power supply to receive supply of power (not illustrated).

Each of the electrode needles 2 is configured to puncture or insert into a treatment target such as a human body to which a high-frequency treatment is performed, and output high-frequency power to the treatment target. Each of the electrode needles 2 is configured to have a needle tube shape that can puncture the human body or the like, and can input a medicine or the like via the electrode needle 2. Each of the electrode needles 2 is electrically connected to each of the electrode connectors T of the high-frequency treatment device 4 via an electrode needle cable C. Each of the electrode needles 2 is configured such that most of the electrode needle 2 excluding a non-insulating portion 21 of a distal end portion is an insulating portion 22 with insulating coating, and the high-frequency current is output from the non-insulating portion 21. A thermocouple connector is provided at a proximal end portion of the electrode needle 2, and the thermocouple connector may be connected to the electrode needle cable C connected to the electrode connector T of the high-frequency treatment device.

Each of the electrode needles 2 is provided with, for example, a thermocouple 23 for measuring a treatment temperature at a distal end portion of a lumen or an inner space thereof, and each of the electrode needles 2 may have a different shape, such as a rod shape, that is inserted into a needle tube or a catheter. In addition, each of the thermocouples 23 may be provided separately from each of the electrode needles 2.

The counter electrode plate 20 is a flat plate-shaped electrode that is disposed by being attached to a skin surface of the human body or the like, which is the treatment target, and is configured to allow a high-frequency current to flow between the counter electrode plate 20 and at least one electrode of the electrode needles 2 inserted therein. In other words, the counter electrode plate 20 is used in a case where the high-frequency current is caused to flow in a so-called monopolar manner. The counter electrode plate 20 is electrically connected to the counter electrode plate connector T0 of the high-frequency treatment device 4 via the counter electrode plate cable C0. The counter electrode plate 20 may have other shapes such as a disk shape in addition to the rectangular flat plate shape. The counter electrode plate 20, the counter electrode plate cable C0, and the counter electrode plate connector T0 may be omitted.

### (Configuration of needle base cover)

The needle base cover 1 as one embodiment of the present invention is for protecting the proximal end portion of the electrode needle 2 (electrically connected to the distal end portion of the electrode needle cable C (specifically, a core wire thereof)). A three-dimensional orthogonal coordinate system (X, Y, and Z) is used for the description of the shape of the needle base cover 1. An X direction corresponds to a "designated direction", a Z direction corresponds to a "first designated direction", and a Y direction corresponds to a "second designated direction".

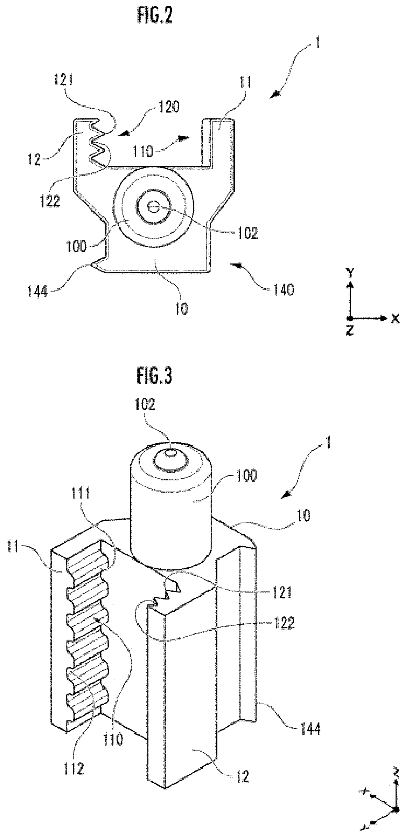

As illustrated in FIGS. 2, 3, and 5, the needle base cover 1 includes a base portion 10, a first clamping portion 11 and a second clamping portion 12 as a pair of clamping portions.

As illustrated in FIG. 2, the base portion 10 is formed in an approximately columnar shape having a cross-sectional shape in which a trapezoid and a rectangle having an upper bottom of the trapezoid as a long side are combined. As illustrated in FIG. 2 (as well as FIGS. 3, 4, 5, and 6), a substantially cylindrical tubular portion 100 having a central axis line parallel to the Z direction (first designated direction) is provided at an upper portion of the base portion 10. A through-hole 102 that communicates with an inner space of the tubular portion 100 and extends in the Z direction (first designated direction), and into which the proximal end portion of the electrode needle 2 is inserted is formed in the base portion 10. In a case where the tubular portion 100 is a part or a configuration element of the base portion 10, the inner space of the tubular portion 100 constitutes a part of the through-hole 102.

The base portion 10, the first clamping portion 11, the second clamping portion 12, and the tubular portion 100 are integrally formed of an elastically deformable synthetic resin. At least two configuration elements of the base portion 10, the first clamping portion 11, the second clamping portion 12, and the tubular portion 100 may be configured as separate members and may be mechanically connected or coupled to each other.

As illustrated in FIG. 2, the first clamping portion 11 and the second clamping portion 12 extend in the Y direction (second designated direction) from a right end portion and a left end portion of a lower bottom of the substantially trapezoidal portion in a top view of the base portion 10, respectively. In a top view (see FIG. 2), longitudinal directions of the first clamping portion 11 and the second clamping portion 12 do not need to be parallel to the Y direction and may be inclined with respect to the Y direction. As illustrated in FIG. 2, the first clamping portion 11 and the second clamping portion 12 are formed in a columnar shape having a substantially rectangular cross-sectional shape and face each other in the X direction (designated direction).

As illustrated in FIG. 3, an inner side portion (a side facing the second clamping portion 12) of the first clamping portion 11 (one clamping portion) has a first uneven structure 110 which extends in a waveform shape in the Z direction (first designated direction) while undulating in the X direction (designated direction). The first uneven structure 110 is configured of (one or) a plurality of first protruding portions 111 (first mount portions) and a plurality of first recessed portions 112 (first valley portions) that are alternately continuous in the Z direction (first designated direction). Each of the first protruding portion 111 and the first recessed portion 112 linearly extends in the Y direction (second designated direction) along an inner side portion of the first clamping portion 11. The number of first protruding portions 111 ("6" in FIG. 3) and the number of first recessed portions 112 ("7" in FIG. 3) may be changed in various ways. The number of the first protruding portions 111 and the number of the first recessed portions 112 may be the same plural.

As illustrated in FIG. 3, both end portions of the first protruding portion 111 having a substantially flat mount apex portion in a front view are formed in a protruding curved surface shape, and the first protruding portion 111 is formed such that a width thereof gradually decreases toward the distal end portion (mount apex portion) in the X direction. As illustrated in FIG. 3, both end portions of the first recessed portion 112 having a substantially flat valley bottom portion in a front view are formed in a recessed curved surface shape, and the first recessed portion 112 is formed such that a width thereof gradually decreases toward a rear end portion (valley bottom portion) in the X direction.

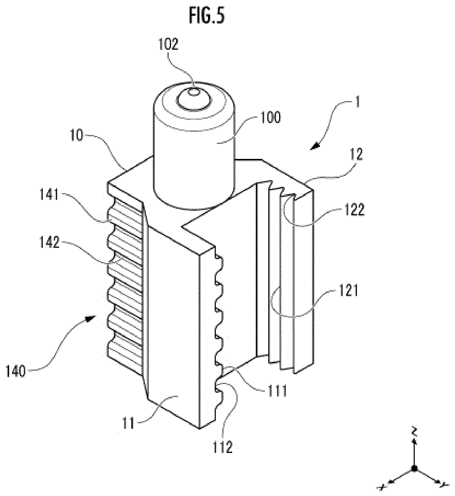

As illustrated in FIGS. 2 and 5, an inner side portion (a side facing the first clamping portion 11) of the second clamping portion 12 (the other clamping portion) has a second uneven structure 120 which extends in a waveform shape in the Y direction (second designated direction) while undulating in the X direction (designated direction). The second uneven structure 120 is configured of (one or) a plurality of second protruding portions 121 (second mount portions) and a plurality of second recessed portions 122 (second valley portions) that are alternately continuous in the Y direction (second designated direction). Each of the second protruding portion 121 and the second recessed portion 122 linearly extends in the Z direction (first designated direction) along the inner side portion of the second clamping portion 12. The number of second protruding portions 121 ("2" in FIG. 2) and the number of second recessed portions 122 ("3" in FIGS. 2 and 5) may be changed in various ways. The number of the second protruding portions 121 and the number of the second recessed portions 122 may be the same plural.

As illustrated in FIG. 2, both end portions of the second protruding portion 121 having a substantially flat mount apex portion in a top view are formed in a protruding curved surface shape, and the second protruding portion 121 is formed such that a width thereof gradually decreases toward the distal end portion (mount apex portion) in the X direction. As illustrated in FIG. 2, both end portions of the second recessed portion 122 having a substantially flat valley bottom portion in a top view are formed in a recessed curved surface shape, and the second recessed portion 122 is formed such that a width thereof gradually decreases toward the rear end portion (valley bottom portion) in the X direction.

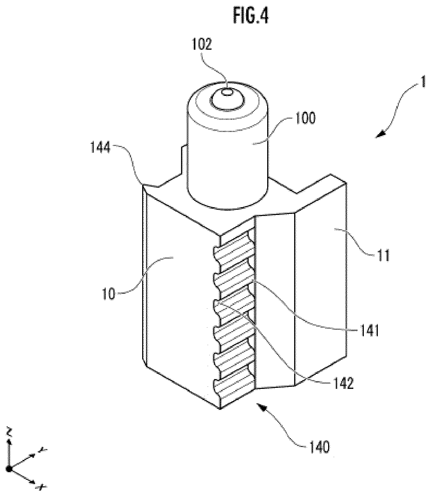

As illustrated in FIGS. 4 and 5, one outer side portion (a right side portion of the substantially rectangular portion in FIG. 2) of base portion 10 has a first sub-uneven structure 140 which extends in a waveform shape in the Z direction (first designated direction) while undulating in the X direction (designated direction). The waveform shape of the first sub-uneven structure 140 corresponds to the waveform shape (see FIG. 3) of the first uneven structure 110. The first sub-uneven structure 140 is configured of (one or) a plurality of first sub-protruding portions 141 (first sub-mount portions) and a plurality of first sub-recessed portions 142 (first sub-valley portions) that are alternately continuous in the Z direction (first designated direction). Each of the first sub-protruding portion 141 and the first sub-recessed portion 142 linearly extends in the Y direction (second designated direction) along one outer side portion of the base portion 10.

As illustrated in FIGS. 4 and 5, in the protruding portion 141 constituting the first sub-uneven structure 140, both end portions of the protruding portion 141 having a substantially flat mount apex portion in a side view are formed in a protruding curved surface shape, and the protruding portion 141 is formed such that a width thereof gradually decreases toward the distal end portion (mount apex portion) in the X direction. As illustrated in FIGS. 4 and 5, in the recessed portion 142 constituting the first sub-uneven structure 140, both end portions of the recessed portion 142 having a substantially flat valley bottom portion in a side view are formed in a recessed curved surface shape, and the recessed portion 142 is formed such that a width thereof gradually decreases toward the rear end portion (valley bottom portion) in the X direction.

As illustrated in FIGS. 4 and 5, an open end portion of the first sub-uneven structure 140 extends in a waveform shape in the Z direction (first designated direction) while undulating in the Y direction (second designated direction). The open end portion of the first sub-uneven structure 140 may extend in the Z direction in a substantially flat shape.

As illustrated in FIGS. 2, 3, and 6, the other outer side portion (the left side portion of the substantially rectangular portion in FIG. 2) of the base portion 10 has a second sub-protruding portion 144 that protrudes in the X direction (the designated direction). As illustrated in FIGS. 3, 4, and 6, the second sub-protruding portion 144 continuously extends in the Z direction (the first designated direction) along the other outer side portion of the base portion 10. As illustrated in FIG. 2, in the second sub-protruding portion 144, both end portions of a substantially flat mount apex portion in a top view are formed in a protruding curved surface shape, and the second sub-protruding portion 144 is formed such that a width thereof gradually decreases toward the distal end portion (mount apex portion) in the X direction.

### (Functions)

According to the high-frequency treatment device 4 including the needle base cover 1 having the above-described configuration and the plurality of electrode needles 2 of which the proximal end portions are protected by the needle base cover 1, the plurality of needle base covers 1 are locked or engaged to be relatively displaceable in each of the Z direction (the first designated direction) and the second designated direction (the Y direction).

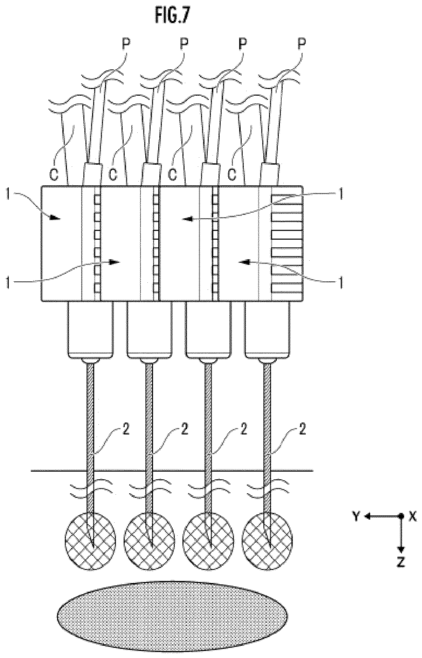

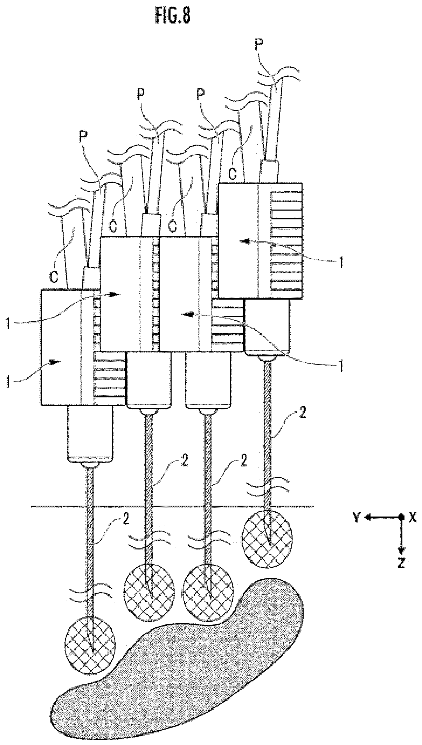

FIG. 7 illustrates a state in which four needle base covers 1 are mutually engaged at the same position (height position) in the Z direction. In the state of FIG. 7, the n1-th (n1 = 1, 2, ..., 6) first sub-protruding portion 141 from the top of the first sub-uneven structure 140 of one needle base cover 1 is engaged with the n1-th first recessed portion 112 from the top of the first uneven structure 110 of the adjacent other needle base cover 1. That is, the first sub-uneven structure 140 of one needle base cover 1 and the first uneven structure 110 of the other needle base cover 1 are engaged with each other over six periods of unevenness. Further, the m1-th (m1 = 1, 2, ..., 6) first protruding portion 111 from the top of the first uneven structure 110 of one needle base cover 1 is engaged with the m1-th first sub-recessed portion 142 from the top of the first sub-uneven structure 140 of the adjacent other needle base cover 1. That is, the first sub-uneven structure 140 of the other needle base cover 1 and the first uneven structure 110 of the one needle base cover 1 are engaged with each other over six periods of unevenness. As a result, the respective height positions of the distal end portions of the four electrode needles 2 are maintained substantially the same, and the four electrode needles 2 are maintained in a posture parallel to the Z direction. In FIGS. 7 and 8, in addition to the electrode needle cable C, the chemical liquid tube P is also inserted into the through-hole 102 of the base portion 10, and the chemical liquid passage of the chemical liquid tube P communicates with the lumen or the inner space of the substantially tubular electrode needle 2.

FIG. 8 illustrates a state in which the needle base cover 1 at the left end is deviated in a +Z direction (downward direction) from the needle base cover 1 on the right side adjacent thereto, and the needle base cover 1 at the right end is deviated in a -Z direction (upward direction) from the needle base cover 1 on the left side adjacent thereto.

In the state of FIG. 8, the n1-th (n1 = 1, 2, and 3) first sub-protruding portion 141 from the top of the first sub-uneven structure 140 of the needle base cover 1 at the left end is engaged with the n1+3-th first recessed portion 112 of from the top of the first uneven structure 110 of the adjacent other needle base cover 1. That is, the first sub-uneven structure 140 of the needle base cover 1 at the left end and the first uneven structure 110 of the needle base cover 1, which is second from the left end are engaged with each other over three periods of unevenness. As a result, the height position of the distal end portion of the electrode needle 2 at the left end is maintained to be lower than the height position of the distal end portion of the electrode needle 2 on the right side adjacent thereof (by three periods of the waveform extending in the Z direction of the first uneven structure 110), and the two electrode needles 2 are maintained in a posture parallel to the Z direction.

In addition, in the state of FIG. 8, the n1-th (n1 = 1, 2, 3, and 4) first sub-protruding portion 141 from the top of the first sub-uneven structure 140 of the needle base cover 1, which is second from the right end, is engaged with the (n1+3)-th first recessed portion 112 from the top of the first uneven structure 110 of the needle base cover 1 at the right end. That is, the first sub-uneven structure 140 of the needle base cover 1 that is second from the right end and the first uneven structure 110 of the needle base cover 1 at the right end are engaged with each other over three periods of unevenness. As a result, the height position of the distal end portion of the electrode needle 2 at the right end is maintained to be higher than the height position of the distal end portion of the electrode needle 2 on the left side adjacent thereof (by three periods of the waveform extending in the Z direction of the first uneven structure 110), and the two electrode needles 2 are maintained in a posture parallel to the Z direction.

From the state of FIG. 7 to the state of FIG. 8, when a practitioner changes the needle base cover 1 at the left end relatively downward with respect to the needle base cover 1 on the right side adjacent thereof by hand, a process in which the first sub-protruding portion 141 of the first sub-uneven structure 140 of the needle base cover 1 at the left end engages with the adjacent first recessed portion 112 by jumping over the first protruding portion 111 from the first recessed portion 112 of the first uneven structure 110 of the needle base cover 1 adjacent to the right is repeated. Similarly, from the state of FIG. 7 to the state of FIG. 8, when the practitioner changes the needle base cover 1 at the right end relatively upward with respect to the needle base cover 1 on the left side adjacent thereof by the hand, a process in which the first sub-protruding portion 141 of the first sub-uneven structure 140 of the second needle base cover 1 from the right engages with the adjacent first recessed portion 112 by jumping over the first protruding portion 111 from the first recessed portion 112 of the first uneven structure 110 of the needle base cover 1 at the right end is repeated. In each of the processes, the practitioner can be made to feel the click sensation through the hand, and can realize that the needle base cover 1 and the electrode needle 2 are relatively deviated or displaced in the Z direction (the first designated direction). Further, the practitioner can recognize a displacement distance based on the click sensation. The same applies to a case of transitioning from the state of FIG. 8 to the state of FIG. 7.

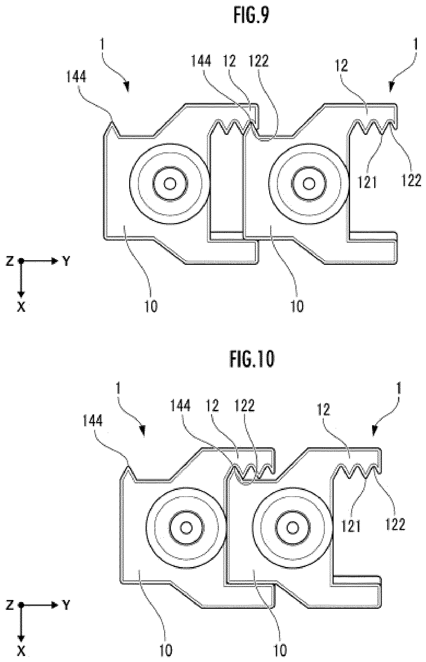

FIG. 9 illustrates a state in which the second sub-protruding portion 144 of the needle base cover 1 on the right side is engaged with the second recessed portion 122 of the second uneven structure 120 of the needle base cover 1 on the left side, which is close to the most distal end portion of the second clamping portion 12. FIG. 10 illustrates a state in which the second sub-protruding portion 144 of the needle base cover 1 on the right side is engaged with the second recessed portion 122 of the second uneven structure 120 of the needle base cover 1 on the left side, which is close to the most rear end portion of the second clamping portion 12. In FIGS. 9 and 10, in addition to the electrode needle cable C, the chemical liquid tube P is also inserted into the through-hole 102 of the base portion 10, and the chemical liquid passage of the chemical liquid tube P communicates with the lumen or the inner space of the substantially tubular electrode needle 2.

From the state of FIG. 9 to the state of FIG. 10, when the practitioner changes the needle base cover 1 on the right side relatively to the needle base cover 1 on the left side in the left direction (-Y direction) with the hand, a process in which the second sub-protruding portion 144 of the needle base cover 1 on right side engages with the adjacent second recessed portion 122 by jumping over the second protruding portion 121 from the second recessed portion 122 of the second uneven structure 120 of the needle base cover 1 on the left side is repeated. In each of the processes, the practitioner can be made to feel the click sensation through the hand, and can realize that the needle base cover 1 and the electrode needle 2 are relatively deviated or displaced in the Y direction (the second designated direction). Further, the practitioner can recognize a displacement distance based on the click sensation. The same applies to a case of transitioning from the state of FIG. 10 to the state of FIG. 9.

### (Other embodiments of present invention)

In the above-described embodiment, the inner side portion of the first clamping portion 11 has the first uneven structure 110 (see FIG. 3), and one outer side portion of the base portion 10 has the first sub-protruding portion 141 (see FIGS. 4 and 5). However, as another embodiment, one outer side portion of the base portion 10 may have the first uneven structure 110, and the inner side portion of the first clamping portion 11 may have the first sub-protruding portion 141. In the above-described embodiment, the first sub-protruding portion 141 is configured of a plurality of protruding portions configuring the first sub-uneven structure 140 (see FIGS. 4 and 5). However, as another embodiment, the first sub-protruding portion 141 may be configured of a single protruding portion, as in the second sub-protruding portion 144.

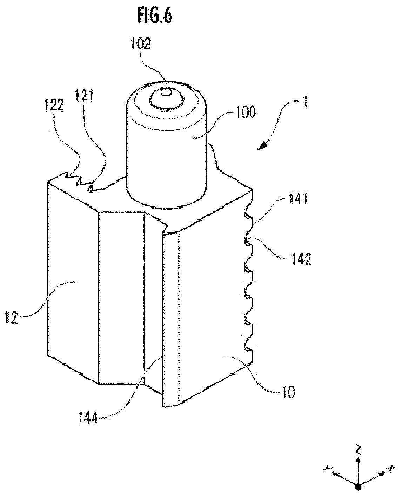

In the above-described embodiment, the inner side portion of the second clamping portion 12 has the second uneven structure 120 (see FIG. 5), and the other outer side portion of the base portion 10 has the second sub-protruding portion 144 (see FIGS. 3 and 6). However, as another embodiment, the other outer side portion of the base portion 10 may have the second uneven structure 120, and the inner side portion of the second clamping portion 12 may have the second sub-protruding portion 144. In the above-described embodiment, the second sub-protruding portion 144 is configured of a single protruding portion (see FIGS. 3 and 6). However, as another embodiment, the second sub-protruding portion 144 may be configured of a plurality of protruding portions configuring the second sub-uneven structure, as in the first sub-protruding portion 141.

### Description of Reference Numerals

1: needle base cover
2: electrode needle
4: high-frequency treatment device
10: base portion
11: first clamping portion
12: second clamping portion
20: counter electrode plate
100: tubular portion
102: through-hole
110: first uneven structure
111: first protruding portion
112: first recessed portion
120: second uneven structure
121: second protruding portion
122: second recessed portion
140: first sub-uneven structure
141: first sub-protruding portion
142: first sub-recessed portion
144: second sub-protruding portion
C: electrode needle cable
T: electrode connector

## Claims

1. A needle base cover for protecting a proximal end portion of an electrode needle that outputs high-frequency power to a treatment target in a state of being punctured to the treatment target, the needle base cover comprising:
a base portion that has a pair of outer side portions which are on opposite sides to each other in a designated direction, and in which a through-hole extending in a first designated direction perpendicular to the designated direction in which the proximal end portion of the electrode needle is inserted is formed; and
a pair of clamping portions that extend from the base portion in a second designated direction perpendicular to each of the designated direction and the first designated direction, and face each other in the designated direction,
wherein one side portion of one outer side portion of the pair of outer side portions and an inner side portion of one clamping portion of the pair of clamping portions of the base portion has a first uneven structure extending in a waveform shape in the first designated direction, and other side portion has a first sub-protruding portion protruding in the designated direction, and
one side portion of the other outer side portion of the pair of outer side portions and an inner side portion of the other clamping portion of the pair of clamping portions of the base portion has a second uneven structure extending in a waveform shape in the second designated direction, and other side portion has a second sub-protruding portion protruding in the designated direction.

2. The needle base cover according to Claim 1,
wherein the first sub-protruding portion is formed such that a width gradually decreases toward a distal end portion in the designated direction, and
the second sub-protruding portion is formed such that a width gradually decreases toward a distal end portion in the designated direction.

3. The needle base cover according to Claim 1,
wherein the first sub-protruding portion is configured of a protruding portion of a first sub-uneven structure that extends in a waveform shape corresponding to the first uneven structure in the first designated direction.

4. The needle base cover according to Claim 1,
wherein the second sub-protruding portion is configured of a protruding portion of a second sub-uneven structure that extends in a waveform shape corresponding to the second uneven structure in the second designated direction.

5. The needle base cover according to Claim 1,
wherein the needle base cover is configured such that a user senses a relative displacement amount in the first designated direction between one needle base cover and another needle base cover through a click sensation when the first sub-protruding portion of the one needle base cover is displaced from one recessed portion to another recessed portion of the first uneven structure of the another needle base cover, and
the needle base cover is configured such that the user senses a relative displacement amount in the second designated direction between the one needle base cover and the another needle base cover through a click sensation when the second sub-protruding portion of the one needle base cover is displaced from one recessed portion to another recessed portion of the second uneven structure of the another needle base cover.

6. A high-frequency treatment device comprising:
a plurality of needle base covers according to any one of Claims 1 to 5;
a plurality of electrode needles of which a proximal end portion is protected by each of the plurality of needle base covers; and
a plurality of electrode cables that are electrically connected to the proximal end portion of each of the plurality of electrode needles,
wherein the high-frequency treatment device is configured such that electrical connection is performed in each of the plurality of electrode needles via the plurality of electrode cables, and high-frequency voltage is applied between the plurality of electrode needles.
